(19) Europäisches Patentamt / European Patent Office / Office européen des brevets

(11) **EP 4 233 856 A1**

(12) **EUROPEAN PATENT APPLICATION**
published in accordance with Art. 153(4) EPC


(43) Date of publication:
**30.08.2023 Bulletin 2023/35**

(21) Application number: **21882047.0**

(22) Date of filing: **20.10.2021**

(51) International Patent Classification (IPC):
***A61K 31/365*** *(2006.01)* ***A61P 35/04*** *(2006.01)*
***A61P 39/00*** *(2006.01)*

(52) Cooperative Patent Classification (CPC):
**A61K 31/365; A61P 35/04; A61P 39/00**

(86) International application number:
**PCT/CN2021/124940**

(87) International publication number:
**WO 2022/083632 (28.04.2022 Gazette 2022/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.10.2020 CN 202011149717**
**14.09.2021 CN 202111084087**



(71) Applicants:
- **Accendatech Co. Ltd.**
  **Tianjin 300102 (CN)**
- **Nankai University**
  **Tianjin 300071 (CN)**

(72) Inventors:
- **CHEN, Yue**
  **Tianjin 300384 (CN)**
- **CAI, Dongpo**
  **Tianjin 300384 (CN)**
- **BAO, Shiqi**
  **Tianjin 300384 (CN)**
- **ZHANG, Xuemei**
  **Tianjin 300384 (CN)**
- **CHEN, Jing**
  **Tianjin 300384 (CN)**
- **GONG, Jianmiao**
  **Tianjin 300384 (CN)**
- **GUO, Jianshuang**
  **Tianjin 300384 (CN)**
- **LI, Jing**
  **Tianjin 300384 (CN)**

(74) Representative: **Gille Hrabal**
**Partnerschaftsgesellschaft mbB**
**Patentanwälte**
**Brucknerstraße 20**
**40593 Düsseldorf (DE)**


(54) **APPLICATION OF SESQUITERPENE LACTONE COMPOUND IN PREPARATION OF DRUGS FOR ALLEVIATING RADIOTHERAPY-INDUCED INJURIES**


(57) An application of a Sesquiterpene lactone compound, and a stereoisomer, isotopic marker, solvate and polymorph thereof, or a pharmaceutically acceptable salt thereof in the preparation of drugs for alleviating radiotherapy-induced injuries. The Sesquiterpene lactone compound is applied to assist radiotherapy, can effectively alleviate the radiotherapy-induced injuries, achieves a protection effect on normal cell tissues of a human body, and has a broad medicinal value.


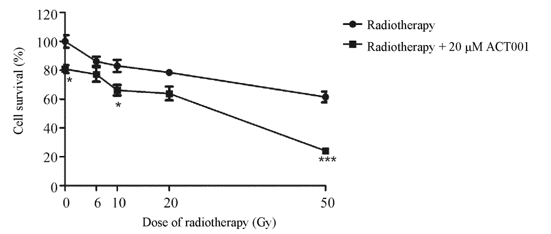


FIG. 1

EP 4 233 856 A1

## Description

The present application claims priority to prior applications of Patent Application No. 202011149717.4 filled with the China National Intellectual Property Administration on October 23, 2020 and entitled with "APPLICATION OF SESQUITERPENE LACTONE COMPOUND IN PREPARATION OF DRUGS FOR ALLEVIATING RADIOTHERAPY-INDUCED INJURIES" and Patent Application No. 202111084087.1 filled with the China National Intellectual Property Administration on September 14, 2021 and entitled with "APPLICATION OF SESQUITERPENE LACTONE COMPOUND IN PREPARATION OF DRUGS FOR ALLEVIATING RADIOTHERAPY-INDUCED INJURIES", which are incorporated herein by reference in their entirety.

## TECHNICAL FIELD

The present disclosure belongs to the field of pharmaceuticals for tumor radiotherapy, and particularly relates to use of a sesquiterpene lactone compound for the manufacturing of a medicament for the alleviation of radiotherapy-induced injuries.

## BACKGROUND

Tumor radiation therapy (abbreviated as radiotherapy) is the treatment of cancer with radiation. The radiotherapy has been in development for over a century. X-rays and radium were used for clinically treating malignant tumors soon after they have been discovered by Roentgen and Madame Curie, respectively. The radiotherapy has been an important local treatment method for malignant tumors until now. Currently, about 70% of cancer patients require the radiotherapy in the process of cancer treatment, and about 40% of cancers can be cured with the radiotherapy. The role and status of the radiotherapy in tumor treatment are increasingly prominent, and the radiotherapy has become one of the main means of treating malignant tumors.

However, in the process of clinical radiotherapy, the radiation rays will inevitably have a certain effect on the normal tissues of a human body, thereby causing certain radiation reactions and injuries. Normal tissue reactions caused by radiation are generally divided into early primary reactions and late secondary reactions. The early radiation reaction generally refers to the injury of tissue cells themselves caused by radiation and possibly concurrent inflammations, such as local mucosal redness and swelling, pain, fester, pseudomembrane formation, and the like caused by acute radiation reactions of oral mucosa and nasal mucosa; acute dry or wet radiation reactions of the skin, and the like. The late radiation reaction refers to the occlusion of small blood vessels and fibrosis of connective tissue caused by radiation, such as dry mouth caused by hyposecretion of glands, fibrosis and contraction of lung, skin and subcutaneous tissues, and the like, which affects the function of tissues and organs. Severe radiation injuries can lead to radiation paraplegia, brain necrosis, osteonecrosis, intestinal necrosis, and the like. Clinical studies have shown that radiation causes some chemical reactions in the body: the production of over-acidified substances, the accumulation into the liver, the liberation of free radicals, and the occurrence of allergic reactions. These factors contribute to symptoms such as lassitude, loss of appetite, vomiting, nausea, and the like. These symptoms usually appear several days after radiotherapy. Individual patients are radiation-sensitive, and when the radiation dose is just half or less, they will develop malaise and have a decrease in white blood cells (near or below normal values, as shown in blood routine examination). These symptoms usually appear 2 to 3 weeks after radiotherapy, and are dominated by a decrease in white blood cells and platelets. Due to the long-term toxicity of radiotherapy, including the occurrence of primary secondary tumors and infertility, patients subjected to radiotherapy need to be protected from the side effects of radiotherapy that can cause further injuries.

Metastatic brain tumors refer to tumor cells that are metastasized to the intracalvairum from other parts of the body. Carcinomas, sarcoma, and melanomas can all metastasize to the intracalvairum. The route and site of metastasis are related to the location of the primary tumor. For example, lung cancer, breast cancer, and skin cancer mainly metastasize through blood stream and are prone to form multiple metastatic cancers in the brain, while digestive tract carcinoma is more likely to metastasize through lymphatic system and spread in the meninges. Brain metastases from solid tumors are the main cause of morbidity and mortality in cancer patients. Autopsies have shown that approximately 25% of patients who die from tumors have intracranial metastases. Clinical studies have shown that 2/3 patients with intracranial metastatic tumors develop symptoms during their survival time. As the population ages, with a steady increase in morbidity and little change in tumor cure rates, there will be more problems related to intracranial metastatic tumors.

Currently, it has been studied that a low dose of scorpion venom has a significant promoting effect on bone marrow hematopoietic cells of mice. A low dose of scorpion venom polypeptide has the most significant promoting effect on peripheral blood WBCs and bone marrow hematopoietic cells of mice. The prevention and treatment of the scorpion venom polypeptide and the scorpion venom have a significant promoting effect on the recovery of the spleen weight index of the mice after radiotherapy. It is concluded that the scorpion venom polypeptide and the scorpion venom have a protection effect on the hematopoietic function of the bone marrow of the mice after radiotherapy. It has also been

studied that Beikeneng® can reduce the myelosuppression and cardiopulmonary injury caused by radiotherapy, especially for the elderly patients, and it can activate the coenzyme system of a human body to improve the metabolic level of organism after entering into cells, and play a certain protection role in radiotherapy. However, these two methods cannot inhibit intracranial metastatic tumors.

## SUMMARY

In order to solve the problems described above in the prior art, the present disclosure provides use of a sesquiterpene lactone compound, and a stereoisomer, an isotopically labeled compound, a solvate and a polymorph thereof or a pharmaceutically acceptable salt thereof for the manufacturing of a medicament for the alleviation of radiotherapy-induced injuries.

According to an embodiment of the present disclosure, the sesquiterpene lactone compound is a micheliolide derivative.

According to an embodiment of the present disclosure, the sesquiterpene lactone compound has the following structure:

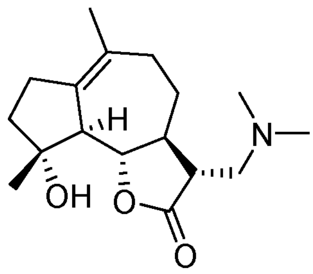

.

According to an embodiment of the present disclosure, the pharmaceutically acceptable salt includes an acid addition salt formed from the sesquiterpene lactone compound and the following inorganic acids: e.g., hydrochloric acid, hydrofluoric acid, hydrobromic acid, hydroiodic acid, sulfuric acid, pyrosulfuric acid, phosphoric acid or nitric acid; or an acid addition salt formed from the sesquiterpene lactone compound and the following organic acids: e.g., formic acid, acetic acid, acetoacetic acid, pyruvic acid, trifluoroacetic acid, propionic acid, butyric acid, caproic acid, enanthic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, hemisulfuric acid or thiocyanic acid.

It will be understood by those skilled in the art that the term "isotopically labeled compound" includes, but is not limited to, the compounds disclosed herein that are labeled by isotopes of hydrogen, carbon, nitrogen, oxygen, fluorine, sulfur and chlorine (e.g., $^{2}H$, $^{3}H$, $^{13}C$, $^{14}C$, $^{15}N$, $^{18}O$, $^{17}O$, $^{18}F$, $^{35}S$ and $^{36}Cl$). The isotopically labeled compound disclosed herein can be used for the determination of the tissue distribution of compounds and prodrugs and metabolites thereof; preferred isotopes for such determinations include $^{3}H$ and $^{14}C$. In addition, in certain cases, substitution with relatively heavy isotopes (e.g., deuterium ($^{2}H$ or D)) can result in greater metabolic stability, which provides therapeutic advantages, for example increased *in vivo* half-life or reduced dose requirements. The isotopically labeled compound disclosed herein can generally be prepared by substituting a non-isotopically-labeled reagent with an isotopically-labeled reagent according to the method described herein.

In some embodiments, the isotopically labeled compound includes a deuterate of the sesquiterpene lactone compound.

According to an embodiment of the present disclosure, the pharmaceutically acceptable salt is selected from the following structure:

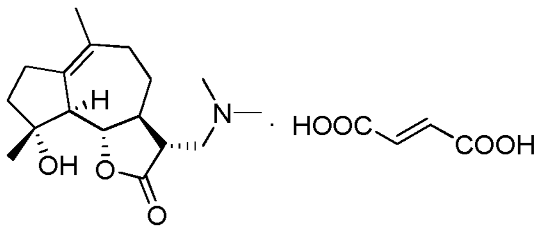

.

According to an embodiment of the present disclosure, the radiotherapy includes radiotherapy for any tumors including solid tumors and metastatic tumors.

The "solid tumor" refers to tumors and/or metastatic tumors (wherever applicable) other than lymphomas, such as tumors of the brain or other central nervous systems (e.g., meningioma, encephaloma, spinal cord tumor, cranial neuroma, and tumors in other sites of the central nervous system, e.g., glioblastomas or medulloblastomas); head and/or neck cancer; breast tumor; tumors of the circulatory system (e.g., tumors of the heart, mediastinum and pleura, and other organs within the thorax, hemangiomas, and tumors related to vascular tissue); tumors of the secretory system (e.g., kidney, pelvis, ureter, bladder, other and unspecified urinary organs); gastrointestinal tumors (e.g., esophagus, stomach, small intestine, colon, colorectum, rectosigmoid junction, rectum, anus, anal canal), and tumors involving the liver and intrahepatic bile ducts, gallbladder, other and unspecified sites of the biliary tract, pancreas and other digestive organs; tumors of the head and neck; tumors of the oral cavity (lips, tongue, gums, floor of mouth, palate and other sites of the oral cavity, parotid gland and other sites of salivary gland, tonsil, oropharynx, nasopharynx, pyriform sinus, hypopharynx and other sites of lips, oral cavity and pharynx); tumors of the reproductive system (e.g., vulva, vagina, cervix, corpus uteri, uterus, ovary, and other sites of the female reproductive organs, placenta, penis, prostate, testis, and other sites of the male reproductive organs); tumors of the respiratory tract (e.g., nasal and middle ear, paranasal sinuses, larynx, trachea, bronchi and lungs, e.g., small cell lung cancer or non-small cell lung cancer); tumors of the skeletal system (e.g., bone and articular cartilage of the extremities, osteoarticular cartilage and other sites); skin tumors (e.g., malignant melanoma of the skin, non-melanoma skin cancer, basal cell carcinoma of the skin, squamous cell carcinoma of the skin, mesothelioma, Kaposi's sarcoma); and tumors involving other tissues, including peripheral and vegetative nervous system, connective and soft tissues, retroperitoneum and peritoneum, eyes and adnexa, thyroid, adrenal glands and other endocrine glands, and related structures, secondary and unspecified malignant lymph node tumors, secondary malignant tumors of the respiratory and digestive systems, and secondary malignant tumors of other sites.

The "metastatic tumor" refers to a metastatic tumor of the primary organ or tissue and/or any other site, regardless of the location of the tumor and/or metastatic tumor.

According to an embodiment of the present disclosure, the radiotherapy-induced injuries include various adverse side effects to the body caused by the radiotherapy, including but not limited to: (1) nasopharyngeal, oral mucosal and skin injuries; (2) cardiac radiation injuries; (3) radiation pneumonia; (4) bone marrow radiation injuries; (5) brain radiation injuries, radiation proctitis, bladder injuries, and the like.

According to an embodiment of the present disclosure, the radiotherapy-induced injuries are radiation injuries caused by radiation therapy, including but not limited to, X-ray-induced radiation injuries, heavy ion radiation-induced radiation injuries, and the like. The X-ray-induced radiation injuries and heavy ion radiation-induced radiation injuries include radiation injuries caused by low linear energy transfer (low-LET) or high linear energy transfer (high-LET) X-ray and radiation injuries caused by heavy ion radiation, respectively. According to a preferred embodiment of the present disclosure, the radiotherapy-induced injuries are radiotherapy-induced injuries of metastatic brain tumors. Therefore, the present disclosure provides use of a sesquiterpene lactone compound for the manufacturing of a medicament for the alleviation of radiotherapy-induced injuries of a metastatic brain tumor. According to another preferred embodiment of the present disclosure, included is use of the sesquiterpene lactone compound for the manufacturing of a medicament for the alleviation of radiotherapy-induced injuries of a metastatic brain tumor by inhibiting the proliferation activity of a cell strain of glioblastoma.

The present disclosure further provides use of the sesquiterpene lactone compound or the pharmaceutically acceptable salt thereof for the manufacturing of a medicament with a protection effect on chemoradiotherapy-induced injuries.

According to another preferred embodiment of the present disclosure, the sesquiterpene lactone compound and pharmaceutically acceptable auxiliary materials are formulated into a medicament.

According to another preferred embodiment of the present disclosure, the medicament is a liquid, gaseous, solid or semisolid formulation.

According to another preferred embodiment of the present disclosure, the medicament is an injectable formulation.

According to another preferred embodiment of the present disclosure, the medicament is an oral formulation.

According to another preferred embodiment of the present disclosure, the oral formulation is a capsule formulation.

According to another preferred embodiment of the present disclosure, the oral formulation is a pill formulation.

Beneficial Effects

(1) In the present disclosure, it is found that the sesquiterpene lactone compounds, particularly with the ACT001 structure, can alleviate radiotherapy-induced injuries and effectively protect normal cell tissues from being affected or injured in the process of radiotherapy.

(2) In the present disclosure, it is found that the sesquiterpene lactone compounds can further alleviate the radiotherapy-induced injuries of a metastatic brain tumor by inhibiting the proliferation activity of a cell strain of glioblastoma.

## BRIEF DESCRIPTION OF THE DRAWINGS

FIG. 1 is a plot showing the effect of ACT001 at different radiation doses on the survival rate of U118-MG cells according to Example 1 of the present disclosure;

FIG. 2 is a plot showing the effect of ACT001 on the survival time of mice subjected to radiotherapy according to Example 1 of the present disclosure;

FIG. 3 is a plot showing the change in body weight of mice before radiotherapy with ACT001 according to Example 1 of the present disclosure;

FIG. 4 is a comparative plot showing the effects of ACT001 combined with or without radiotherapy on body weight of mice according to Example 1 of the present disclosure.

FIG. 5 is a comparative graph showing the effect of ACT001 combined with radiotherapy on the survival of lung cancer cells (H226) and normal lung epithelial cells (BEAS2B) according to Example 1 of the present disclosure.

FIG. 6 shows the effect of ACT001 combined with radiotherapy on the survival of SY5Y cells according to Example 1 of the present disclosure.

FIG. 7 shows the neuronal apoptosis of brain tissue taken 14 days after the whole brain radiotherapy (2 Gy×4, single dose of 4 Gy) of BALB/c mice.

## DETAILED DESCRIPTION

The present disclosure is described in detail below with reference to the drawings, and the description in this section is only exemplary and explanatory and should not be construed as limiting the scope of the present disclosure in any way. Furthermore, features of examples described in this document and various examples may be combined by those skilled in the art accordingly, based on the description in this document.

Example 1

The sesquiterpene lactone compound used for the manufacturing of a medicament for the alleviation of radiotherapy-induced injuries of a metastatic brain tumor has the following structural formula:

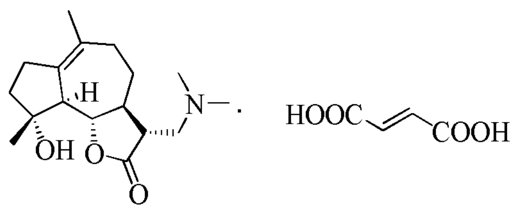

,

which is also known as ACT001, with a chemical name of (3A,3aS,9A,9aS,9bS)-3-((dimethylamino)methyl)-9-hydroxy-6,9-dimethyl-3,3a,4,5,7,8,9,9a-octahydroazuleno[4,5-*b*]furan-2(9b*H*)-one fumarate, and is a micheliolide derivative.

The pharmacological and pharmacodynamic experiment are as follows:

## I. *In Vitro* Experiment - Anti-Proliferation Activity of ACT001

### 1. Experimental objective

To investigate the effect of ACT001 at different radiation doses on the anti-proliferation activity of a U118-MG cell strain

### 2. Experimental materials

2.1 Test cell

U118-MG human brain astroblastoma cell strain, purchased from Cell Bank of Chinese Academy of Sciences, Shanghai Institutes for Biological Sciences

2.2 Experimental instruments and reagents

Table 1. Experimental instruments and reagents

| Name | Model | Source |
|---|---|---|
| Double single-sided purification workbench | SW-CJ-2FD | Suzhou Purification Equipment Co., Ltd. |
| Carbon dioxide incubator | 3111 | Thermo Fisher Scientific |
| Inverted biological microscope XDS-3 | XDS-3 | Optika |
| Microplate reader | MμLtiscan FC | Thermo Fisher Scientific |
| 4°C display cabinet | SC350 | Henan Xinfei Electric Group Co., Ltd. |
| Liquid nitrogen biological container | Model YDS-65-216 | Chengdu Golden Phoenix Liquid Nitrogen Container Co., Ltd. |
| Pipettor | 10 μL/200 μL/1000 μL | Da Long Xing Chuang Experimental Instrument (Beijing) Co., Ltd. |
| Culture dish | 60/90/100 mm | Thermo Fisher Scientific |
| Vortex mixer | XH-C | Changzhou Lang Yue Instrument Manufacturing Co., Ltd. |
| High-speed refrigerated centrifuge | 75002440 | Thermo Fisher Scientific |
| PBS powder | P1010 | Beijing Solarbio Science & Technology Co., Ltd. |
| DMEM | 11965-092 | Gibco |
| Pancreatin | 25200072 | Gibco |
| DMSO | D8371 | Beijing Solarbio Science & Technology Co., Ltd. |
| 96-well plate | 3599 | Corning |
| FBS | P150407 | PAN |
| CCK-8 | CA1210 | Beijing Solarbio Science & Technology Co., Ltd. |

### 3. Experimental procedures

The culture medium for U118-MG was DMEM, and the cells were cultured in a cell incubator at 37 °C with 5% $CO_2$.

Recover of U118-MG cells: a cryopreservation tube was taken from liquid nitrogen, placed in water bath at 37 °C to thaw the cryopreservation solution, and centrifuged at 1000 rpm for 5 min. The supernatant was discarded, and the cells were resuspended in a complete culture medium and cultured under the condition of 37 °C, 5% $CO_2$.

When 70-80% of the bottom of the culture flask was covered by the cells, the culture medium was discarded by pipetting. The cells were washed with 2-3 mL of PBS buffer and digested with 1-2 mL of 0.25% pancreatin (just enough to cover the bottom of the flask). Immediately after the cell became round after being cultured in the incubator for a period of time, a complete culture medium was added to stop the digestion, and the cell suspensions after the digestion were combined and centrifuged at 1000 rpm for 5 min. The supernatant was discarded, and the cells were

resuspended in a complete culture medium, gently pipetted, and counted. The cell density was adjusted to 2500 cells/well, and the cells were seeded into a 96-well plate at 90 μL/well.

Dosing was performed one day after the cells were seeded. Each plate was divided into a blank group, a negative control group and 10 compound groups, with 4-6 duplicate wells set for each group. 10 μL of the compound was added to each well. 3 h later, each well received a corresponding radiation dose. The cells were cultured at 37 °C for 24 h. After that, the culture medium was discarded, a corresponding fresh culture medium was added, and the plate was incubated for 48 h. At the end of incubation, 10 μL of CCK-8 solution (taking care not to create air bubbles in the wells, which would affect the OD readings) was added to each well, and the plate was incubated in the incubator for 1-4 h. The OD value at 450 nm was determined with a microplate reader.

The data were processed with an EXCEL software to solve inhibition rates at different concentrations, and $IC_{50}$ values were obtained by fitting with SPSS. The mean and standard deviation of the $IC_{50}$ values obtained three times were solved by SPSS.

$$\text{Inhibition rate \%} = \{1 - (\text{OD value of compound well} - \text{OD value of blank well})/(\text{OD value of control well} - \text{OD value of blank well})\} \times 100\%$$

**4. Experimental results**

The radiation was performed at the Institute of Radiation Medicine, Chinese Academy of Medical Sciences. The results are shown below.

Table 2. Inhibition rate (%) at different radiation doses

| Dose | 0 Gy | 6 Gy | 10 Gy | 20 Gy | 50 Gy |
|---|---|---|---|---|---|
| ACT001-0 μM | | 13.78 | 16.92 | 21.51 | 38.42 |
| ACT001-20 μM | 19.17 | 22.83 | 33.81 | 36.07 | 75.62 |

As can be seen from Table 2, with the increase of radiation dose, the inhibition rates of both ACT001-0 μM and ACT001-20 μM groups are increased. When the radiation dose was 10 Gy, the 20 μM group showed a significant increase in the inhibition rate compared with the 0 μM group, and when the radiation dose was 50 Gy, the 20 μM group showed a very significant increase in the inhibition rate compared with the 0 μM group. As can be seen from FIG. 1, ACT001-20 μM can significantly enhance the inhibition effect of the radiation group on the proliferation of the U118-MG cells.

II. *In Vitro* Experiment - Effect of ACT001 on Normal Cells

1. Experimental objective

To investigate the selective radiotherapy protection effect of ACT001 on normal cells

2. Experimental materials

2.1 Test cell

H226 human squamous lung carcinoma cell strain and BEAS2B human normal lung epithelial cell strain, purchased from Cell Bank of Chinese Academy of Sciences, Shanghai Institutes for Biological Sciences

2.2 Experimental instruments and reagents

Table 3. Experimental instruments and reagents

| Name | Model | Source |
|---|---|---|
| Double single-sided purification workbench | SW-CJ-2FD | Suzhou Purification Equipment Co., Ltd. |
| Carbon dioxide incubator | 3111 | Thermo Fisher Scientific |
| Inverted biological microscope XDS-3 | XDS-3 | Optika |
| Microplate reader | MμLtiscan FC | Thermo Fisher Scientific |
| 4 °C display cabinet | SC350 | Henan Xinfei Electric Group Co., Ltd. |
| Liquid nitrogen biological container | Model YDS-65-216 | Chengdu Golden Phoenix Liquid Nitrogen Container Co., Ltd. |
| Pipettor | 10 μL/200 μL/1000 μL | Da Long Xing Chuang Experimental Instrument (Beijing) Co., Ltd. |
| Culture dish | 60/90/100 mm | Thermo Fisher Scientific |
| Vortex mixer | XH-C | Changzhou Lang Yue Instrument Manufacturing Co., Ltd. |
| High-speed refrigerated centrifuge | 75002440 | Thermo Fisher Scientific |
| PBS powder | P1010 | Beijing Solarbio Science & Technology Co., Ltd. |
| DMEM | 11965-092 | Gibco |
| Pancreatin | 25200072 | Gibco |
| DMSO | D8371 | Beijing Solarbio Science & Technology Co., Ltd. |
| 96-well plate | 3599 | Corning |
| FBS | P150407 | PAN |
| CCK-8 | CA1210 | Beijing Solarbio Science & Technology Co., Ltd. |

3. Experimental procedures

The culture medium for H226 and BEAS2B cells was DMEM, and the cells were cultured in a cell incubator at 37 °C with 5% $CO_2$.

Recover of H226 and BEAS2B cells: a cryopreservation tube was taken from liquid nitrogen, placed in water bath at 37 °C to thaw the cryopreservation solution, and centrifuged at 1000 rpm for 5 min. The supernatant was discarded, and the cells were resuspended in a complete culture medium and cultured under the condition of 37 °C, 5% $CO_2$.

When 70-80% of the bottom of the culture dish was covered by the cells, the culture medium was discarded. The cells were washed with 2-3 mL of PBS buffer and digested with 1 mL of 0.25% pancreatin (just enough to cover the bottom of the flask). Immediately after the cell became round after being cultured in the incubator for a period of time, a complete culture medium was added to stop the digestion, and the cell suspensions after the digestion were combined and centrifuged at 1000 rpm for 5 min. The supernatant was discarded, and the cells were resuspended in a complete culture medium, gently pipetted, and counted. The cell density was adjusted to 2500 cells/well, and the cells were seeded into a 96-well plate at 90 μL/well.

Dosing was performed 24 h after the cells were seeded. Each plate was divided into a blank group, a negative control group and an ACT001-10 μM administration group, with 6 duplicate wells set for each group. 10 μL of the compound was added to each well, and the radiation doses were 0 Gy, 2 Gy, 4 Gy, and 6 Gy. The radiation was performed 1 h after dosing, and the cells were cultured for 48 h after the radiation. 10 μL of CCK-8 solution (taking care not to create air bubbles in the wells, which would affect the OD readings) was added to each well, and the plate was incubated in the incubator for 1-4 h. The OD value at 450 nm was determined with a microplate reader.

4. Experimental results

As can be seen from FIG. 5, radiation can induce death of H226 cells and BEAS2B cells, and ACT001 can significantly reduce radiotherapy-induced injuries of BEAS2B cells, but has no protection effect on H226 tumor cells.

III. *In Vitro* Experiment - Alleviation of Radiation Injuries by ACT001

1. Experimental objective

To investigate the alleviation effect of ACT001 on radiation-induced neuronal cell death

2. Experimental materials

2.1 Test cell

SY5Y human neuroblastoma cell strain, purchased from Cell Bank of Chinese Academy of Sciences, Shanghai Institutes for Biological Sciences

2.2 Experimental instruments and reagents

Table 4. Experimental instruments and reagents

| Name | Model | Source |
|---|---|---|
| Double single-sided purification workbench | SW-CJ-2FD | Suzhou Purification Equipment Co., Ltd. |
| Carbon dioxide incubator | 3111 | Thermo Fisher Scientific |
| Inverted biological microscope XDS-3 | XDS-3 | Optika |
| Microplate reader | MμLtiscan FC | Thermo Fisher Scientific |
| 4 °C display cabinet | SC350 | Henan Xinfei Electric Group Co., Ltd. |
| Liquid nitrogen biological container | Model YDS-65-216 | Chengdu Golden Phoenix Liquid Nitrogen Container Co., Ltd. |
| Pipettor | 10 μL/200 μL/1000 μL | Da Long Xing Chuang Experimental Instrument (Beijing) Co., Ltd. |
| Culture dish | 60/90/100 mm | Thermo Fisher Scientific |
| Vortex mixer | XH-C | Changzhou Lang Yue Instrument Manufacturing Co., Ltd. |
| High-speed refrigerated centrifuge | 75002440 | Thermo Fisher Scientific |
| PBS powder | P1010 | Beijing Solarbio Science & Technology Co., Ltd. |
| DMEM | 11965-092 | Gibco |
| Pancreatin | 25200072 | Gibco |
| DMSO | D8371 | Beijing Solarbio Science & Technology Co., Ltd. |
| 96-well plate | 3599 | Corning |
| FBS | P150407 | PAN |
| CCK-8 | CA1210 | Beijing Solarbio Science & Technology Co., Ltd. |

3. Experimental procedures

The culture medium for SY5Y cells was DMEM, and the cells were cultured in a cell incubator at 37 °C with 5% $CO_2$.

Recover of SY5Y cells: a cryopreservation tube was taken from liquid nitrogen, placed in water bath at 37 °C to thaw the cryopreservation solution, and centrifuged at 1000 rpm for 5 min. The supernatant was discarded, and the cells were resuspended in a complete culture medium and cultured under the condition of 37 °C, 5% $CO_2$.

When 70-80% of the bottom of the culture dish was covered by the cells, the culture medium was discarded. The cells were washed with 2-3 mL of PBS buffer and digested with 1 mL of 0.25% pancreatin (just enough to cover the bottom of the flask). Immediately after the cell became round after being cultured in the incubator for a period of time, a complete culture medium was added to stop the digestion, and the cell suspensions after the digestion were combined and centrifuged at 1000 rpm for 5 min. The supernatant was discarded, and the cells were resuspended in a complete

culture medium, gently pipetted, and counted. The cell density was adjusted to 2500 cells/well, and the cells were seeded into a 96-well plate at 90 μL/well.

Dosing was performed 24 h after the cells were seeded. Each plate was divided into a negative control group and ACT001 administration groups at doses of 1.25 μM, 2.5 μM, 5 μM, and 10 μM, with 6 duplicate wells set for each group. 10 μL of the compound was added to each well, and the radiation doses were 0 Gy, 2 Gy, 4 Gy, 6 Gy, 8 Gy, and 10 Gy. The radiation was performed 1 h after dosing, and the cells were cultured for 48 h after the radiation. 10 μL of CCK-8 solution (taking care not to create air bubbles in the wells, which would affect the OD readings) was added to each well, and the plate was incubated in the incubator for 1-4 h. The OD value at 450 nm was determined with a microplate reader.

4. Experimental results

As can be seen from FIG. 6, with the increase of radiation dose, the injuries of SY5Y cells caused by radiation are gradually increased, but ACT001 can significantly reduce the injuries of SY5Y cells caused by radiation. Since SY5Y cells are used as a cell model of normal neurons, it is presumed that ACT001 can reduce the injuries of brain neurons caused by radiation and exert a certain protection effect against radiotherapy.

IV. *In Vivo* Experiment - Effect of ACT001 on Survival of Mice Subjected to Radiotherapy

**1. Experimental objective**

To investigate whether ACT001 can improve the survival rate or prolong the survival time of BALB/C mice subjected to radiotherapy

**2. Experimental materials**

2.1 Test sample

Name: ACT001; supplier: Tianjin Accendatech Technology Co., Ltd.;
appearance: white powder; purity: 99.38%;
product batch number: C10553-16.

2.2 Radiotherapy device

Radiotherapy device from Tianjin Medical University Cancer Institute & Hospital

2.3 Experimental animal and feeding conditions

Adult BALB/c mice, 18-19 g, 8 weeks, female, SPF (Beijing) Biotechnology Co., Ltd., animal certificate number: SCXK (Beijing) 2016-0002. The test animals were bred in the common environment of the experimental animal center of the Institute of Radiation Medicine, Chinese Academy of Medical Sciences, with 5 animals in each cage. The padding was autoclaved special padding for mice, and the mice were fed with special sterilized feed, and were free to drink purified water. In the animal laboratory, the temperature was kept at about 25 °C, the relative humidity was kept at 40%-70%, and the illumination was provided for 12 h daily.

2.4 Radiation dose selection

In a preliminary experiment of radiation dose exploration, mice were subjected to radiation at 8 Gy, 10 Gy, 12 Gy, and 14 Gy, respectively. All animals died 18 days after the radiation. However, the animals in the 8 Gy group survived longer than the other groups, so the radiation dose of 8 Gy was selected for the formal experiment.

**3. Experimental procedures and drug treatment**

3.1 Experimental procedures

(1) The lethal dose of a single radiation of mice was determined through a preliminary experiment;
(2) the mice were randomly grouped by the body weight;
(3) ACT001 was pre-administered for 17 consecutive days, and the state of the animals was observed and the body weight was measured daily;
(4) the mice in groups (3) and (4) were subjected to radiation at a lethal dose after 17 days of the administration, and on the day of radiation, ACT001 was administered 1 h before the radiation;
(5) ACT001 was continuously administered for five weeks after the radiation, and the survival state of the animals was observed and the body weight was measured daily. The survival rate and survival time of mice in each group were calculated.

3.2 Grouping regimen

(1) Blank control group
(2) ACT001-200 mg/kg group
(3) Radiotherapy-8 Gy group
(4) ACT001-200 mg/kg + radiotherapy-8 Gy group

A total of 4 groups were set in the experiment, with 10 animals in each group.

3.3 Administration method

ACT001 (dissolved in normal saline) was administered intragastrically once daily, with an interruption once a week;
an equal volume of normal saline was administered intragastrically to the blank control group.

**4. Data processing**

Data were expressed as $\bar{x} \pm s$; statistical analysis among groups was performed using the t-test program in EXCEL software, and the logrank chi-square test method was used to evaluate whether there was statistical significance in each group of dead animals.

**5. Results**

ACT001 was administered for 17 consecutive days after grouping, and the animals were in good state with stable body weight. On day 18 after ACT001 administration, radiotherapy was performed in the radiotherapy-8 Gy group and ACT001-200 mg/kg + radiotherapy-8 Gy group. In the radiotherapy-8 Gy group, animal death occurred on day 24 after the radiotherapy, and median survival was reached on day 27; all animals in this group died by day 32, with a survival rate of 0%. In the ACT001-200 mg/kg + radiotherapy-8 Gy group, the animals were in good state, and animal death occurred on day 15; no further animal death occurred after the death of the third animal in the group by day 31, with a survival rate of 70%. The survival time of the ACT001-200 mg/kg + radiotherapy-8 Gy group was significantly prolonged compared with that of the radiotherapy control-8 Gy group, and was statistically different ($P < 0.01$). The results show that ACT001 at a dose of 200 mg/kg can significantly improve the survival rate and prolong the survival time of mice subjected to radiation.
The survival curves (Kaplan-Meier curves) and data of BABL/c mice are detailed in Table 3, FIG. 2, FIG. 3 and FIG. 4.

Table 3. Protection effect of ACT001 on mice subjected to radiotherapy (repeated experiment) ( $\bar{x} \pm s$)

| Group | Route of administration | Number of animals (mice) | | Body weight (g) | | (after radiotherapy) Median survival time (day) | Survival rate (%) |
|---|---|---|---|---|---|---|---|
| | | Start | End | Start | End | | |
| Blank control group | p.o.×49 | 10 | 10 | 19.0±0.5 | 21.5±1.2 | | 100 |

(continued)

| Group | Route of administration | Number of animals (mice) | | Body weight (g) | | (after radiotherapy) Median survival time (day) | Survival rate (%) |
|---|---|---|---|---|---|---|---|
| | | Start | End | Start | End | | |
| Radiotherapy-8 Gy group | systemic radiotherapy × 1 | 10 | 0 | 19.0±0.5 | | 27 | 0 |
| ACT001-200mg/kg | p.o.×49 | 10 | 10 | 19.0±0.5 | 19.1±3.0 | | 100 |
| ACT001-200 mg/kg + radiotherapy-8 Gy group | p.o. × 49 + systemic radiotherapy × 1 | 10 | 7 | 19.0±0.5 | 18.5±2.9 | | 70 |

FIG. 2 is a plot showing the effect of ACT001 on the survival time of mice subjected to radiotherapy. Note: no animals died in both the blank control group and the ACT001-200 mg/kg group, and the two curves were superimposed. "**" indicates $p < 0.01$ compared with the radiotherapy-8 Gy group.

FIG. 3 is a plot showing the change in body weight of mice before radiotherapy.

FIG. 4 is a plot showing the effect of ACT001 combined with radiotherapy on body weight of mice.

As can be seen from Table 3 and FIGs. 2-4, ACT001 at a dose of 200 mg/kg can improve the survival rate and prolong the survival time of mice subjected to radiation. In the experiment of protection effect on mice subj ected to radiotherapy, ACT001 can significantly prolong the survival time of animals and improve the survival rate of the animals.

V. *In Vivo* Experiment - effect of ACT001 on radiotherapy-induced injuries of mice

1. Experimental objective

To investigate the effect of ACT001 on radiation-induced neuronal apoptosis of mice

2. Experimental materials

2.1. Test sample

Name: ACT001; supplier: Tianjin Accendatech Technology Co., Ltd.;
appearance: white powder; purity: 99.38%;
product batch number: C10553-16.

2.2 Radiotherapy device

Radiotherapy device from the Institute of Radiation Medicine, Chinese Academy of Medical Sciences.

2.3 Experimental animal and feeding conditions

Adult BALB/c mice, 18-19 g, 8 weeks, female, SPF (Beijing) Biotechnology Co., Ltd., animal certificate number: SCXK (Beijing) 2019-0010. The test animals were bred in the barrier environment of the experimental animal center of the Institute of Radiation Medicine, Chinese Academy of Medical Sciences, with 5 animals in each cage. The padding was autoclaved special padding for mice, and the mice were fed with special sterilized feed, and were free to drink purified water. In the animal laboratory, the temperature was kept at about 25 °C, the relative humidity was kept at 40%-70%, and the illumination was provided for 12 h daily. 3. Experimental procedures and compound treatment

3.1 Administration method

ACT001 (dissolved in sterile normal saline) was administered intragastrically once daily; an equal volume of

sterile normal saline was administered to the model control group;
The compound for each group was prepared freshly prior to use.

3.2 Experimental grouping

(1) Model control group
(2) Radiotherapy control group (4 Gy)
(3) ACT001-200 mg/kg group
(4) ACT001-200 mg/kg +4 Gy group
(5) Radiotherapy low-dose control group (2 Gy)
(6) ACT001-200 mg/kg +2 Gy group

10 animals in each group, 60 animals in total.

3.3 Experimental procedures

The mice were randomly grouped, with 10 mice in each group. ACT001-200 mg/kg was administered 48 h before radiation, then the mice were anesthetized and subjected to radiation treatment. The 4 Gy groups were subjected to single brain radiation; the 2 Gy groups were subjected to continuous radiation for 4 days, once daily, and the model control group was anesthetized at an equal dose on the day of radiation. On day 14 after the radiation, whole brains were fixed in 4% paraformaldehyde for Tunel staining to determine the injuries of brain neurons caused by radiation.

4. Data processing

Statistical analysis among groups was performed using the t-test program in EXCEL software.

5. Experimental results

As can be seen from FIG. 7, compared with the model control group, both the multiple-radiation 2 Gy group and the single-radiation 4 Gy group can induce apoptosis of neurons in the cerebral cortex; compared with the radiation-only groups, the radiation combined with ACT001-200 mg/kg can significantly reduce the apoptosis of neurons, which indicates that ACT001 can protect normal brain tissues and reduce radiation-induced neuronal damage of mice.
The example described above is a preferred embodiment of the present disclosure, which, however, is not intended to limit the embodiments of the present disclosure. Any other changes, modifications, substitutions, combinations, and simplifications can be made without departing from the spirit and principle of the present disclosure, and should be the equivalent replacements and included in the protection scope of the present disclosure.

## Claims

1. Use of a sesquiterpene lactone compound, and a stereoisomer, an isotopically labeled compound, a solvate or a polymorph thereof or a pharmaceutically acceptable salt thereof in the manufacturing of a medicament for the alleviation of radiotherapy-induced injuries, wherein the sesquiterpene lactone compound is as shown below:

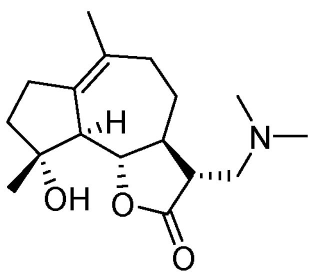

.

2. The use according to claim 1, wherein the pharmaceutically acceptable salt is selected from the following structure:

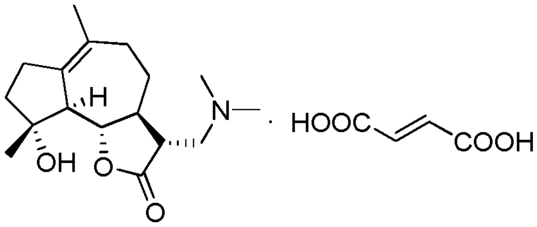

.

3. The use according to claim 1 or 2, wherein the radiotherapy comprises solid tumors and metastatic tumors.

4. The use according to any one of claims 1-3, wherein the radiotherapy-induced injuries are radiation injuries caused by radiation therapy, e.g., X-ray-induced radiation injuries and heavy ion radiation-induced radiation injuries.

5. The use according to any one of claims 1-4, wherein the sesquiterpene lactone compound alleviates radiotherapy-induced injuries of a metastatic brain tumor by inhibiting the proliferation activity of a cell strain of glioblastoma.

6. The use according to claim 1, wherein the sesquiterpene lactone compound and pharmaceutically acceptable auxiliary materials are formulated into a medicament.

7. The use according to claim 6, wherein the medicament is a liquid, gaseous, solid or semisolid formulation.

8. The use according to claim 6, wherein the medicament is an injectable formulation.

9. The use according to claim 6, wherein the medicament is an oral formulation.

10. The use according to claim 6, wherein the medicament is a capsule formulation.

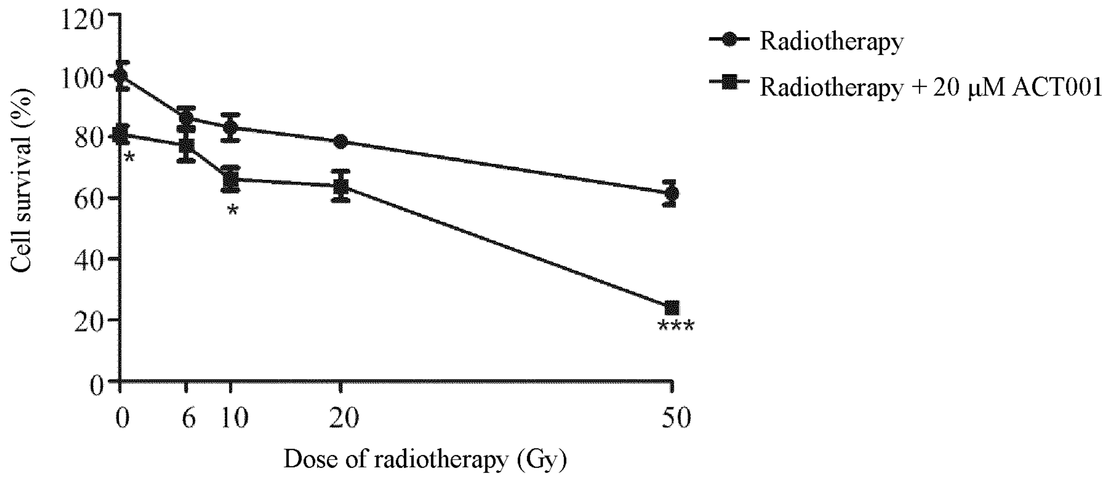
120
100
80
60
40
20
0
Cell survival (%)
0
6
10
20
50
Dose of radiotherapy (Gy)
*
*
***
Radiotherapy
Radiotherapy + 20 μM ACT001

FIG. 1

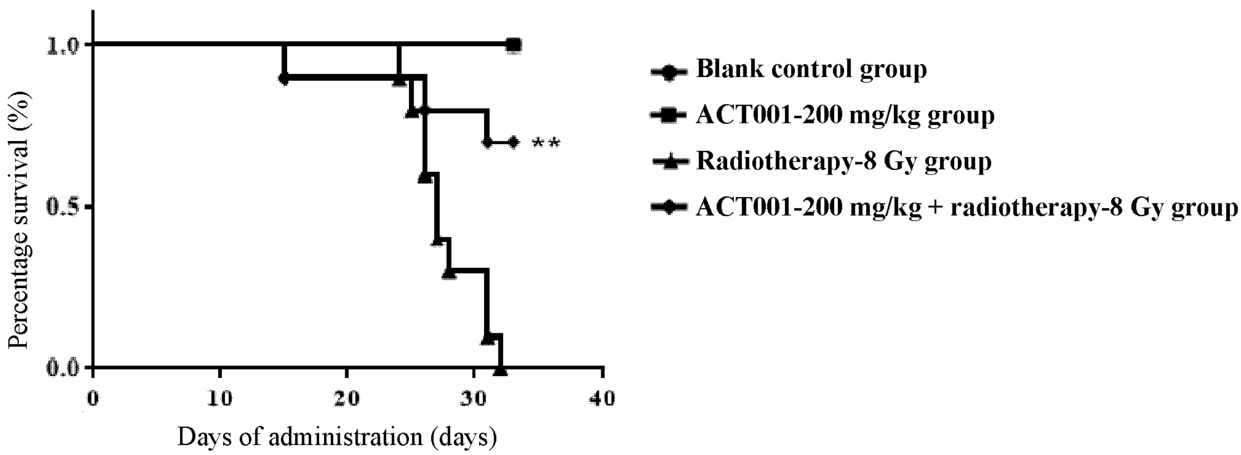
1.0
0.5
0.0
Percentage survival (%)
0
10
20
30
40
Days of administration (days)
**
Blank control group
ACT001-200 mg/kg group
Radiotherapy-8 Gy group
ACT001-200 mg/kg + radiotherapy-8 Gy group

FIG. 2

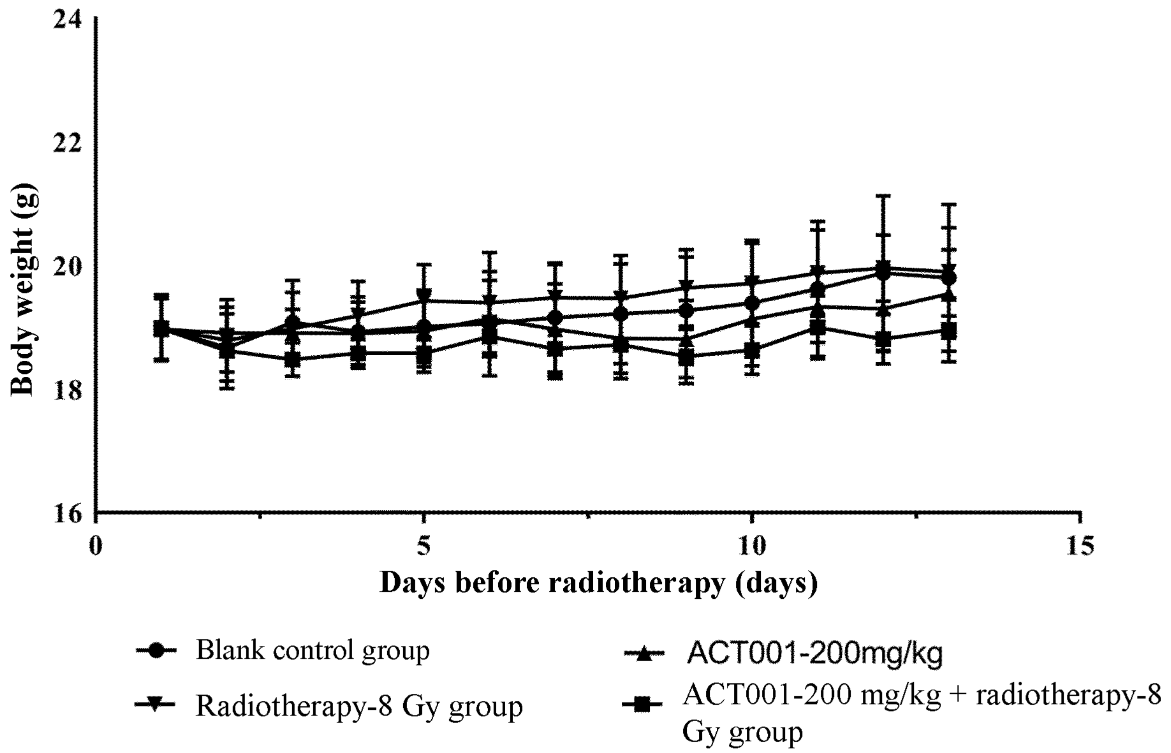
24
22
20
18
16
Body weight (g)
0
5
10
15
Days before radiotherapy (days)
Blank control group
ACT001-200mg/kg
Radiotherapy-8 Gy group
ACT001-200 mg/kg + radiotherapy-8 Gy group

FIG. 3

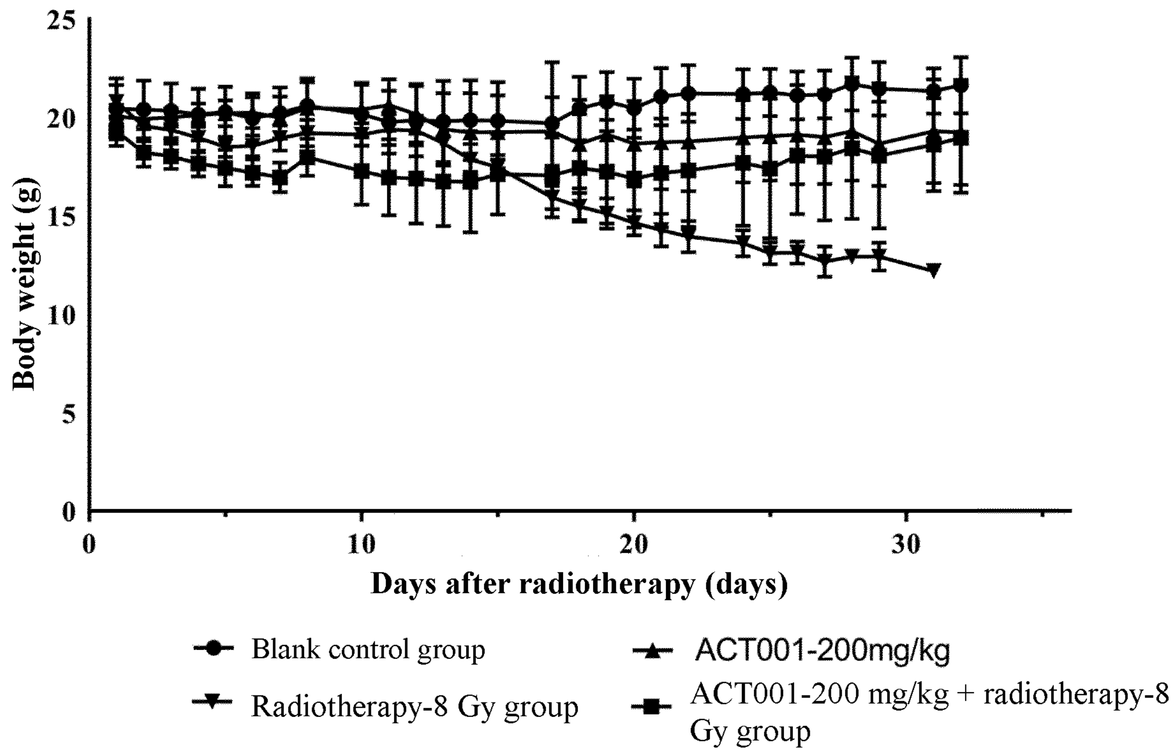
25
20
15
10
5
0
Body weight (g)
0
10
20
30
Days after radiotherapy (days)
Blank control group
ACT001-200mg/kg
Radiotherapy-8 Gy group
ACT001-200 mg/kg + radiotherapy-8 Gy group

FIG. 4

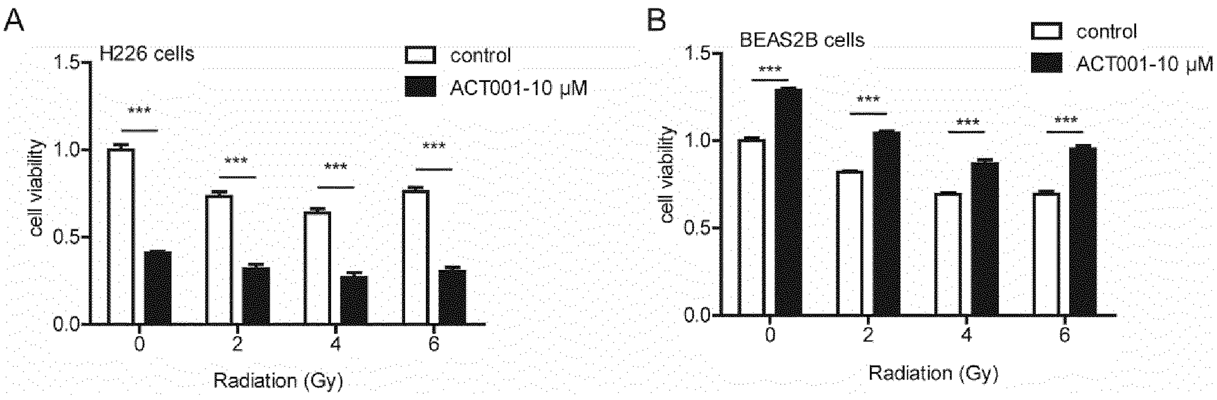


FIG. 5

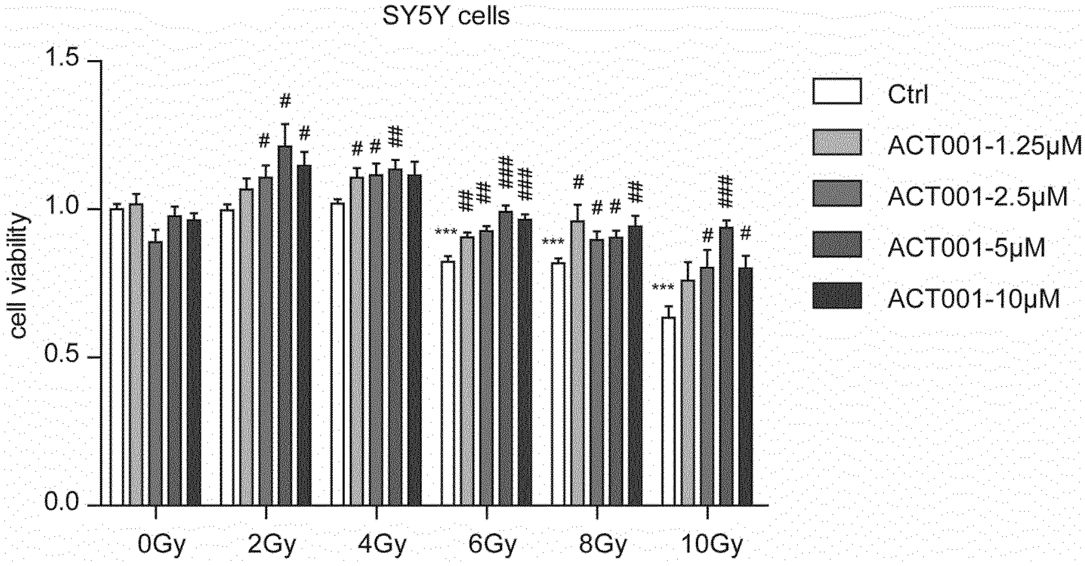


FIG. 6

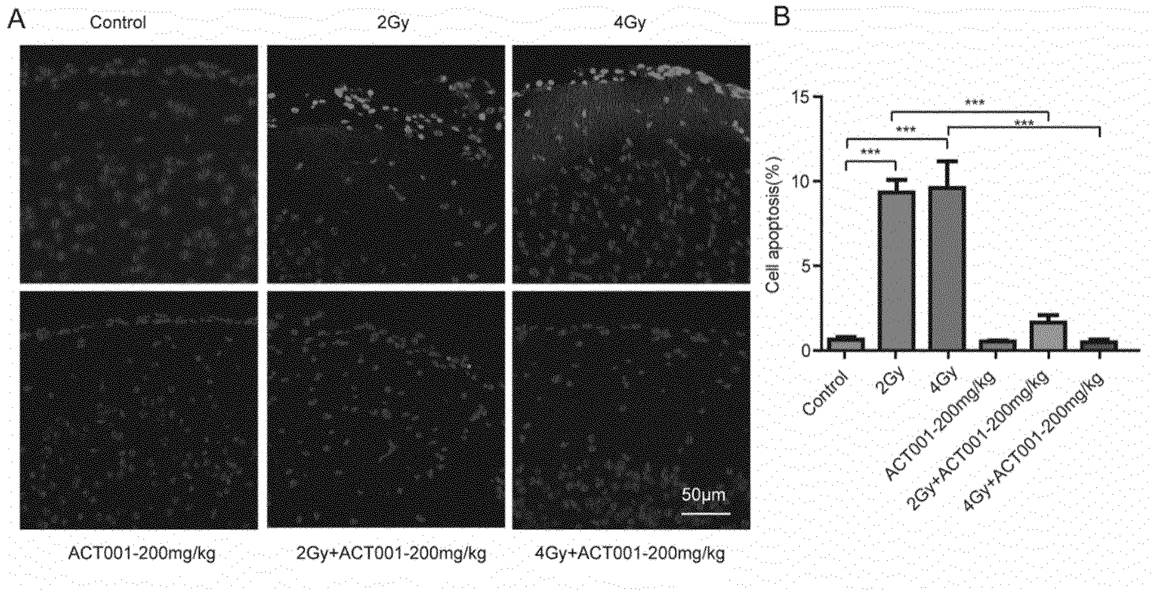


FIG. 7

**INTERNATIONAL SEARCH REPORT**

International application No. **PCT/CN2021/124940**

**A. CLASSIFICATION OF SUBJECT MATTER**

A61K 31/365(2006.01)i; A61P 35/04(2006.01)i; A61P 39/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, DWPI, SIPOABS, CNTXT, EPTXT, USTXT, WOTXT, CNKI, PUBMED-CHEM, BING, STNext, 百度学术, 读秀: 天津尚德药缘, 南开大学, 陈悦, 蔡冬坡, 鲍世琦, 张雪梅, 陈静, 龚建苗, 郭建爽, 李静, ACT 001, 1582289-91-5, ACT001, MICHELIOLIDE, dimethylaminoparthenolide, parthenolide, 小白菊内酯, 含笑内酯, 倍半萜内酯, RADIATION, RADIOTHERAPY, radiation-induced, stat, 放射, 放疗, 辐射, 损失, 损伤, injury, damage, Prognosis, 预后, structure search

**C. DOCUMENTS CONSIDERED TO BE RELEVANT**

| Category* | Citation of document, with indication, where appropriate, of the relevant passages | Relevant to claim No. |
|---|---|---|
| Y | US 2019314324 A1 (THE UNIVERSITY OF CHICAGO) 17 October 2019 (2019-10-17)<br>description, paragraphs [0037]-[0040], [0045]-[0048], [0153]-[0157] | 1-10 |
| Y | WO 2010059245 A2 (THE JOHNS HOPKINS UNIVERSITY) 27 May 2010 (2010-05-27)<br>description page 1 paragraph 2 from the bottom to page 2 paragraph 5, page 9 paragraph 2, page 11 table 1A | 1-10 |
| Y | TONG, Luqing et al.,. "ACT001 reduces the expression of PD-L1 by inhibiting the phosphorylation of STAT3 in glioblastoma,"<br>*Theranostics,* Vol. 10, No. 13, 01 May 2020 (2020-05-01),<br>pp. 5943-5956 | 1-10 |
| Y | MOREL, Katherine L. et al.,. "Parthenolide Selectively Sensitizes Prostate Tumor Tissue to Radiotherapy while Protecting Healthy Tissues In Vivo,"<br>*Radiation Research,* Vol. vol. 187, No. 5, 03 March 2017 (2017-03-03),<br>pp. 501-512 | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 December 2021** | **18 January 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/124940**

**C. DOCUMENTS CONSIDERED TO BE RELEVANT**

| Category* | Citation of document, with indication, where appropriate, of the relevant passages | Relevant to claim No. |
|---|---|---|
| Y | ZHOU, Guijuan et al.,. "Ionizing radiation modulates vascular endothelial growth factor expression through STAT3 signaling pathway in rat neonatal primary astrocyte cultures," *Brain and Behavior,* Vol. 10, No. 4, 27 February 2020 (2020-02-27), pp. 1-12 | 1-10 |
| Y | XI, Xiaonan et al.,. "ACT001, a novel PAI-1 inhibitor, exerts synergistic effects in combination with cisplatin by inhibiting PI3K/AKT pathway in glioma," *Cell Death and Disease,* Vol. 10, No. 10, 07 October 2019 (2019-10-07), pp. 1-17 | 1-10 |
| Y | DERASKA, Peter V. et al.,. "NF-κB inhibition by dimethylaminoparthenolide radiosensitizes non-small-cell lung carcinoma by blocking DNA double-strand break repair," *Cell Death Discovery,* Vol. 4, No. 10, 07 February 2018 (2018-02-07), pp. 1-12 | 1-10 |
| Y | MOREL, Katherine L. et al.,. "DMAPT is an effective radioprotector from long-term radiation-induced damage to normal mouse tissues in vivo," *RADIATION RESEARCH,* Vol. 192, No. 2, 16 May 2019 (2019-05-16), pp. 231-239 | 1-10 |
| PY | LICKLITER, Jason D. et al.,. "Phase 1 dose-escalation study of ACT001 in patients with recurrent glioblastoma and other advanced solid tumors," *Journal of Clinical Oncology,* Vol. 39, No. 15 S, 28 May 2021 (2021-05-28), pp. 1-3 | 1-10 |

Form PCT/ISA/210 (second sheet) (January 2015)

**INTERNATIONAL SEARCH REPORT**
**Information on patent family members**

International application No.
**PCT/CN2021/124940**

| Patent document cited in search report | Publication date (day/month/year) | Patent family member(s) | Publication date (day/month/year) |
|---|---|---|---|
| US 2019314324 A1 | 17 October 2019 | US 2020261403 A1 | 20 August 2020 |
| WO 2010059245 A2 | 27 May 2010 | CA 2744388 A1 | 27 May 2010 |
| | | US 2012029071 A1 | 02 February 2012 |
| | | EP 2362770 A2 | 07 September 2011 |
| | | EP 2362770 A4 | 30 May 2012 |
| | | WO 2010059245 A3 | 23 September 2010 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202011149717 A
- WO 202111084087 A